# EUROPÄISCHE PATENTSCHRIFT

(11) **EP 1 273 271 B1**
(45) Veröffentlichungstag und Bekanntmachung des Hinweises auf die Patenterteilung: **15.08.2007**
(21) Anmeldenummer: 02013878.0
(22) Anmeldetag: 22.06.2002
(51) Int. Cl.: A61B 17/17

(54) **Chirurgisches Instrument**
Surgical instrument
Instrument chirurgical

(30) Priorität: 02.07.2001 DE 10131993
(43) Veröffentlichungstag der Anmeldung: 08.01.2003
(73) Patentinhaber: Aesculap AG & Co. KG, 78532 Tuttlingen (DE)
(72) Erfinder: Hilgendorff, Viktor, 15721 Rathenow (DE); Böttiger, Roland, 78604 Rietheim-Weilheim (DE); Saueressig, Thomas, 78532 Tuttlingen (DE)
(74) Vertreter: Hoeger, Stellrecht & Partner Patentanwälte

(56) Entgegenhaltungen:
- EP-A- 0 468 192
- EP-A- 0 617 927
- FR-A- 2 706 279
- US-A- 4 465 065
- US-B1- 6 183 477

## Beschreibung

Die Erfindung betrifft ein chirurgisches Instrument zum Einsetzen und Halten einer mindestens zwei Knochenteile verbindenden Knochenplatte, deren Form eine Einführrichtung definiert, in der die Knochenplatte an die zu verbindenden Knochenteile herangeführt wird, mit einem Halteabschnitt, mit mindestens einem an diesem angeordneten Kupplungselement zum lösbaren Verbinden desselben mit mindestens einem an der Knochenplatte vorgesehenen Verbindungselement, wobei das Kupplungselement derart angeordnet ist, daß in einer Einführstellung, in der das Instrument mit der Knochenplatte verbunden ist, das Instrument die Knochenplatte im wesentlichen entgegengesetzt zur Einführrichtung verlängert.

Instrumente der eingangs beschriebenen Art werden zum Einführen von Knochenplatten verwendet, die beispielsweise zur Schienung von pertrochanteren Femurfrakturen oder kondylären Femur- und Tibia-Frakturen dienen. Die Knochenplatten werden üblicherweise durch eine kleine Inzision unter die Haut und durch den Muskel hindurch geschoben. Dabei spaltet die Platte das Muskelgewebe.

Ein gattungsgemäßes Instrument ist beispielsweise aus der EP 0 617 927 B1 bekannt. Es wird im wesentlichen quer zur Längsrichtung der Knochenplatte, die die Einführrichtung vorgibt, an dieser verschraubt. Dies muß insbesondere dann spielfrei und paßgenau der Fall sein, wenn das Instrument gleichzeitig auch als Zielgerät zum Einsetzen von Knochenschrauben oder Drähten dient.

Als nachteilig erweist sich bei der in der EP 617 927 B1 offenbarten Ausgestaltung, daß die seitliche Befestigung des Instruments an der Knochenplatte eine Inzision erforderlich macht, die größer ist, als es für die Knochenplatte allein notwendig wäre. Aus der EP 0 468 192 A2 ist eine Einführungs- und Zielvorrichtung beschrieben, die mit einer Befestigungsschraube mit einer Osteosyntheseplatte verbindbar ist.

Es ist daher Aufgabe der vorliegenden Erfindung, ein chirurgisches Instrument der eingangs beschriebenen Art so zu verbessern, daß das Einführen der Knochenplatte in einen menschlichen Körper erleichtert wird, eine möglichst geringe Inzision zum Einsetzen der Knochenplatte erforderlich wird und auf einfache Weise eine Verbindung zwischen der Knochenplatte und dem Instrument hergestellt werden kann.

Diese Aufgabe wird bei einem chirurgischen Instrument der eingangs beschriebenen Art erfindungsgemäß dadurch gelöst, daß an dem mindestens einen Kupplungselement mindestens ein in der Einführstellung in eine an der Knochenplatte angeordnete, korrespondierende Ausnehmung eintauchender Vorsprung vorgesehen ist.

Diese Ausgestaltung hat den Vorteil, daß eine Inzision ausreichend ist, durch die die Knochenplatte eingeführt werden kann, denn das Instrument steht nicht seitlich von der Knochenplatte ab, was eine aufgrund des Querschnitts des Instruments und seiner Anordnung größere Inzision erforderlich machen würde. Darüber hinaus wird das Einführen der Knochenplatte erleichtert, denn ein Operateur kann aus der Orientierung des Instruments im wesentlichen direkt auf die Orientierung der Knochenplatte schließen, da beide Teile in etwa in die gleiche Richtung weisen. Im wesentlichen entgegengesetzt zur Einführrichtung bedeutet, daß das Instrument und die Einführrichtung einen Winkel von maximal 30° einschließen. Dadurch, daß an dem mindestens einen Kupplungselement mindestens ein in der Einführstellung in eine an der Knochenplatte angeordnete, korrespondierende Ausnehmung eintauchender Vorsprung vorgesehen ist, kann auf einfach herzustellende Weise eine kraftschlüssige Verbindung zwischen der Knochenplatte und dem Instrument hergestellt werden.

Grundsätzlich kann das Instrument allein zum Einsetzen und Halten der Knochenplatte dienen, allerdings ist es vorteilhaft, wenn das Instrument einen Zielgerätabschnitt mit mindestens einer eine Längsachse aufweisenden Zielgerätdurchbrechung umfaßt und wenn der mindestens einen Zielgerätdurchbrechung mindestens eine an der Knochenplatte vorgesehene Durchbrechung mit identischer Längsachse zugeordnet ist. Das Instrument kann dadurch insgesamt als Zielgerät zum Fixieren der Knochenplatte an den Knochenteilen dienen. Dabei gibt die Längsachse der mindestens einen Zielgerätdurchbrechung dem Operateur die Richtung für eine an den Knochenteilen zu setzende Bohrung vor, deren Längsachse mit der der mindestens einen Durchbrechung an der Knochenplatte zusammenfällt. Somit werden Fehlbohrungen vermieden.

Um eine Position des Instruments relativ zur Knochenplatte eindeutig festzulegen, ist vorteilhafterweise ein Spannglied zum Verspannen der Knochenplatte mit dem Instrument in der Einführstellung vorgesehen. Damit kann eine spielfreie Verbindung der beiden Teile in der Einführstellung hergestellt werden.

Um zusätzlich eine Relativposition zwischen der Knochenplatte und dem Instrument in einer Ebene quer zur Einführrichtung festzulegen, kann vorzugsweise ein mit einem an der Knochenplatte angeordneten Zentrierelement zusammenwirkendes Zentrierglied zum Festlegen einer definierten relativen Kupplungsstellung zwischen der Vorrichtung und der Knochenplatte in der Einführstellung vorgesehen sein. Durch das Zentrierglied wird die Relativposition zwischen Knochenplatte und Instrument eindeutig festgelegt, was insbesondere bei Verwendung des Instruments als Zielgerät Abweichungen bei zu setzenden Bohrungen vermeiden hilft.

Dabei kann es vorteilhaft sein, wenn das Zentrierglied einen in Längsrichtung des Halteabschnitts festleg- und auf die Knochenplatte hin bewegbaren Druckstift umfaßt und wenn das Zentrierglied ein zum Zentrierelement im wesentlichen korrespondierendes freies Ende aufweist. Durch den Druckstift, der gegenüber dem Instrument beispielsweise geklemmt oder geschraubt werden kann, werden die Knochenplatte und das Instrument in Einführrichtung fest gegeneinander gedrückt und durch das zum Zentrierelement korrespondierende Zentrierglied wird die Position des Instruments relativ zur Knochenplatte mindestens in einer Ebene quer zur Einführrichtung genau vorgegeben. Das Zentrierglied kann beispielsweise eine kegelförmige Spitze und das Zentrierelement kann eine kegelförmige, korrespondierende Ausnehmung umfassen. Eine umgekehrte Anordnung von Spitze und Ausnehmung an den beiden Teilen wäre ebenfalls möglich.

Vorzugsweise umfaßt das Spannglied das Zentrierelement. Dadurch wird die Zahl der beweglichen Teile am Instrument reduziert. Das Spannglied hat somit eine Doppelfunktion. Es kann gleichzeitig mit dem Verspannen der Knochenplatte mit dem Instrument deren relative Lage eindeutig festgelegt werden.

Grundsätzlich kann vorgesehen sein, daß das mindestens eine Kupplungselement mit dem mindestens einen Verbindungselement kraftschlüssig verbindbar ist. Durch diese Ausgestaltung werden keine weiteren Teile zur Fixierung der Knochenplatte am Instrument benötigt.

Bei einer weiteren bevorzugten Ausführungsform der Erfindung kann vorgesehen sein, daß das mindestens eine Kupplungselement mit dem mindestens einen Verbindungselement formschlüssig verbindbar ist. Dadurch kann eine Verbindung der beiden Teile miteinander hergestellt werden, die getrennt davon durch ein weiteres Verbindungselement gesichert werden kann.

Besonders vorteilhaft ist es dabei, wenn der mindestens eine Vorsprung durch eine quer zur Einführrichtung verlaufende Rastkante und wenn die Ausnehmung durch eine Nut gebildet werden. Durch Eingreifen der Rastkante in die Nut entsteht eine Rastverbindung zwischen Knochenplatte und Instrument, durch die ohne weitere Verbindungsmittel eine Haltekraft vom Instrument auf die Knochenplatte übertragen werden kann.

Bei einer anderen bevorzugten Ausführungsform der Erfindung kann vorgesehen sein, daß der mindestens eine Vorsprung durch einen quer zur Einführrichtung verlaufenden Stift und daß die Ausnehmung durch eine Durchbrechung der Knochenplatte gebildet wird. Aufgrund dieser Ausgestaltung kann die Knochenplatte nur in Längsrichtung des Stifts auf das Instrument geschoben werden. Ihre Relativposition zum Instrument in Einführrichtung kann aber eindeutig vorgegeben werden.

Grundsätzlich kann vorgesehen sein, daß der in der Einführstellung an die Knochenplatte angrenzende Halteabschnitt in einem Übergangsbereich einen Querschnitt aufweist, der höchstens so groß wie der größte Querschnitt der Knochenplatte ist. Dadurch wird zusätzlich sichergestellt, daß die zum Einführen der Knochenplatte erforderliche Inzision auch zum Einführen des Instruments in den menschlichen Körper ausreicht. Außerdem können so zusätzliche Vorsprünge, Kanten oder Ähnliches im Übergangsbereich zwischen der Knochenplatte und dem Einsetzinstrument vermieden werden, die das Einsetzen der Knochenplatte beim Durchdringen des Körpergewebes erschweren.

Der Halteabschnitt könnte grundsätzlich einteilig ausgeführt sein. Vorteilhaft ist es jedoch, wenn der Halteabschnitt zwei Haltearme umfaßt, die sich in Längsrichtung des Halteabschnitts erstrecken und wenn die Haltearme freie Enden aufweisen. Dies ermöglicht eine Verbindung zwischen Knochenplatte und Instrument derart, daß zusätzlich zu einer in Einführrichtung übertragenen Kraft vom Instrument auf die Knochenplatte Kräfte quer zur Einführrichtung vom Instrument auf die Knochenplatte übertragen werden können, wodurch die Knochenplatte noch sicherer am Instrument gehalten wird.

Besonders vorteilhaft ist es dabei, wenn das mindestens eine Kupplungselement an einem oder an beiden freien Enden der Haltearme angeordnet ist. Dadurch können beispielsweise auch zwei Kupplungselemente und damit auch zwei Verbindungen zwischen der Knochenplatte und dem Instrument hergestellt werden.

Grundsätzlich können die Haltearme biegesteif ausgestaltet sein. Günstig ist es aber, wenn die Haltearme quer zu ihrer Längsachse biegeelastisch geformt sind. Durch Verbiegen der Haltearme quer zu deren Längsrichtung, können diese vorgespannt werden, wodurch auf einfache Weise eine Kraft in entgegengesetzter Richtung ausgeübt werden kann, die zum Halten der Knochenplatte am Einsetzinstrument dient.

Der Zielgerätabschnitt könnte direkt am Halteabschnitt angeordnet werden. Gemäß einer bevorzugten Ausführungsform der Erfindung ist mindestens ein Distanzelement vorgesehen und das Distanzelement den Zielgerätabschnitt und den Halteabschnitt verbindend angeordnet. Dadurch wird es möglich, das Instrument individuell an Patienten mit unterschiedlichem Körpervolumina anzupassen. Der Zielgerätabschnitt kann dadurch in stets geringstem Abstand von der Knochenplatte an diese angeordnet werden, bei Bedarf jedoch in einem größeren Abstand.

Die nachfolgende Beschreibung bevorzugter Ausführungsformen der Erfindung dient im Zusammenhang mit der Zeichnung der näheren Erläuterung. Es zeigen:
- Figur 1:: Eine perspektivische Gesamtansicht eines Instruments mit daran angeordneter Knochenplatte beim Verschrauben derselben mit einem Femurknochen;
- Figur 2:: eine Querschnittsansicht längs Linie 2 - 2 in Figur 1;
- Figur 3:: eine Ansicht in Richtung des Pfeils A in Figur 2;
- Figur 4:: eine alternative Ausführungsform des Instruments mit einer alternativen Form einer Knochenplatte; und
- Figur 5:: eine Seitenansicht in Richtung des Pfeils B in Figur 4.

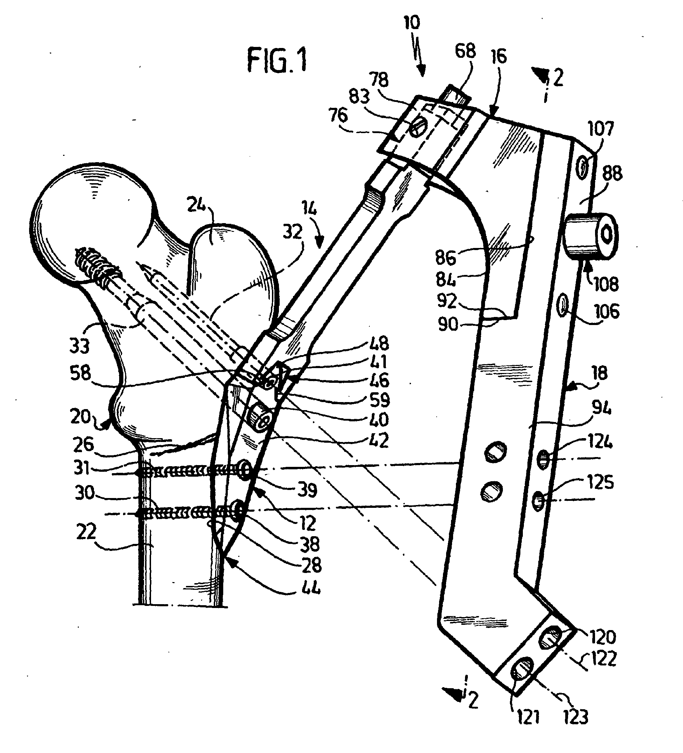

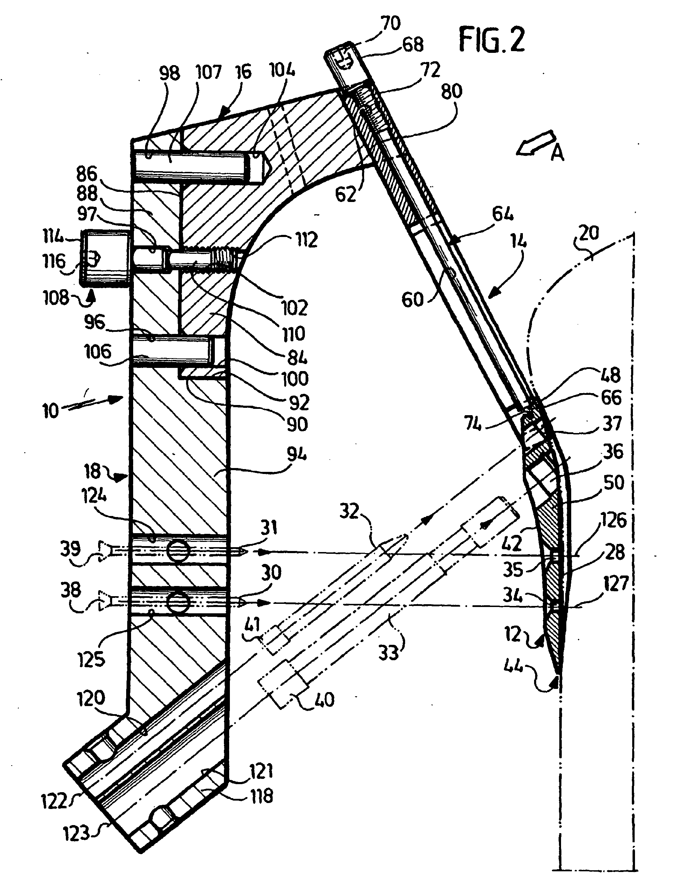

In den Figuren 1 und 2 ist ein insgesamt mit dem Bezugszeichen 10 versehenes erstes Ausführungsbeispiel eines als Zielgerät ausgestalteten erfindungsgemäßen chirurgischen Instruments dargestellt. Es umfaßt einen eine Knochenplatte 12 haltenden, eine Längsrichtung definierenden Halteabschnitt 14, der über ein Distanzelement 16 paßgenau und spielfrei mit einem Zielgerätabschnitt 18 verbunden ist.

Die Form der Knochenplatte 12 ist an die Form eines Femurknochens 20 im Übergangsbereich zwischen einem Femurschaft 22 und einem Trochanter major 24 angepaßt, um den gebrochenen Femurknochen 20 entlang der Bruchflächen 26 zusammenzuhalten. Eine Anlagefläche 28 der Knochenplatte 12 ist im wesentlichen konvex gekrümmt, und schmiegt sich daher an den Femurknochen 20 an. Fixiert wird die Knochenplatte 12 mit vier Knochenschrauben 30, 31, 32 und 33, die durch dafür vorgesehene, an den Durchmesser der Schäfte der Knochenschrauben 30 bis 33 angepaßte Querbohrungen 34, 35, 36 und 37 der Knochenplatte 12 hindurchgesteckt und so weit in den Femurschaft 22 beziehungsweise Trochanter major 24 eingeschraubt werden, bis Schraubenköpfe 38, 39, 40 und 41 der Knochenschrauben 30 bis 33 an der der Anlagefläche 28 im wesentlichen gegenüberliegenden Anschlagfläche 42 in der Knochenplatte 12 anschlagen.

Die Knochenplatte 12 weist ein im wesentlichen keilförmiges und zugespitztes Einführende 44 sowie ein Kupplungsende 46 auf, an dem ein in einer Seitenansicht trapezförmiger Kupplungszapfen 48 angeordnet ist, und zwar derart, daß beim Übergang in einen Mittelbereich 50 der Knochenplatte 12 zwei parallel verlaufende keilförmige Nute 51 und 52 ausgebildet sind, durch die zwei im wesentlichen quer zu einer Längsrichtung der Knochenplatte 12 verlaufende Schulterflächen 53 und 54 gebildet werden.

Zum Verbinden mit der Knochenplatte 12 weist der Halteabschnitt 14 an seinem distalen Ende eine zum Kupplungszapfen 48 korrespondierende Plattenaufnahme 56 auf, in die der Kupplungszapfen 48 parallel zu der von den Nuten 51 und 52 vorgegebenen Richtung eingeschoben werden kann. Im Bereich ihres offenen Endes weist die Plattenaufnahme 56 zwei Rastkanten 58 und 59 auf, die in die Nute 51 und 52 eingreifen und an den Schulterflächen 53 und 54 anliegen. Dadurch wird eine Relativbewegung zwischen der Knochenplatte 12 und dem Halteabschnitt 14 in Längsrichtung des Halteabschnitts verhindert.

Der Querschnitt des Halteabschnitts 14 im Bereich der Plattenaufnahme 56 entspricht in etwa dem Querschnitt der Knochenplatte 12 in dem an die Nute 51 und 52 angrenzenden Mittelbereich 50. Der Halteabschnitt 14 und die Knochenplatte 12 bilden daher im verbundenen Zustand eine Einheit, die im Übergangsbereich keine Kanten, Vorsprünge oder ähnliches aufweist.

Um eine Relativbewegung quer zur Längsrichtung des Halteabschnitts 14 zu verhindern, weist dieser eine Längsbohrung 60 auf, die an ihrem proximalen Ende mit einem Innengewinde 62 versehen ist. Die Längsbohrung 60 wird von einem eine kegelförmige Spitze 66 aufweisenden Druckstift 64 durchsetzt, der an seinem proximalen Ende einen mit einem Innensechskant 70 versehenen Schraubkopf 68 aufweist, der aus der Längsbohrung 60 herausragt und an den sich ein mit einem Außengewinde 72 versehener kurzer Abschnitt des Druckstifts 64 anschließt.

Am Kupplungszapfen 48 ist ein zur Spitze 66 korrespondierendes, sacklochartiges Zentrierloch 74 angeordnet, in das die Spitze 66 des Druckstifts 64 eintauchen kann, wenn die Knochenplatte 12 mit dem Halteabschnitt 14 verbunden ist. Beim Einschrauben des Druckstifts 64 verspannt sich bei gleichzeitiger Zentrierung der Halteabschnitt 14 mit der Knochenplatte 12, so daß eine spielfreie und genau vorgegebene Position der beiden Teile relativ zueinander eingenommen wird.

Das im wesentlichen quaderförmige proximale Ende 76 des Halteabschnitts 14 wird von zwei Schenkeln 78 und 79 einer U-förmigen Halteabschnittaufnahme 80 des Distanzelements 16 paßgenau umgeben. Mittels zweier quer zur Längsrichtung des Halteabschnitts 14 orientierten Verbindungsschrauben 82 und 83 sind die Schenkel 78 und 79 mit dem Halteabschnitt 14 spielfrei verschraubt.

Das insgesamt im wesentlichen L-förmig gekrümmte Distanzelement 16 umfaßt einen Halteschenkel 84, der eine langgestreckte rechteckige Zielgerätanlagefläche 86 umfaßt, die ganzflächig an einem Befestigungsabschnitt 88 des Zielgerätabschnitts 18 anliegt, ebenso wie eine Stirnfläche 90 des Halteschenkels an einem Rücksprung 92 des Zielgerätabschnitts, der im Übergangsbereich zwischen dem Befestigungsabschnitt 88 und einem Zielabschnitt 94 des Zielgerätabschnitts 18 gebildet wird.

Der Befestigungsabschnitt 88 weist drei Haltebohrungen 96, 97 und 98 auf, deren Längsachsen parallel verlaufen und in Richtung auf den Halteschenkel 84 weisen. Dieser wiederum ist mit einer Querbohrung 100, einer mit einem Innengewinde Haltebohrung 102 und einer Sacklochbohrung 104 versehen, deren Längsachsen beim Anliegen des Halteschenkels 84 am Befestigungsabschnitt 88 mit jeweils einer Längsachse einer der Haltebohrungen 96 bis 98 fluchten.

In den zwei äußeren Haltebohrungen 96 und 98 sind zylinderförmige Stifte 106 und 107 eingesetzt und fest mit dem Befestigungsabschnitt 88 verbunden. Sie dienen als eine Verdrehsicherung zwischen dem Distanzelement 16 und dem Zielgerätabschnitt 18. Um diese fest miteinander zu verspannen, ist eine Spannschraube 108 vorgesehen, deren Schaft 110 an seinem distalen Ende mit einem Außengewindeabschnitt 112 versehen ist, der zum Innengewinde der Haltebohrung 102 korrespondiert. Als Einschraubhilfe ist ein Kopf 114 in der Spannschraube 108 mit einer als Innensechskant ausgestalteten Werkzeugaufnahme 116 versehen.

Zum Zusammenbauen des Zielgeräts 10 werden die Stifte 106 und 107 in die korrespondierende Querbohrung 100 und die Sacklochbohrung 104 eingeschoben und mittels der Spannschraube 108 werden das Distanzelement 16 und der Zielgerätabschnitt 18 miteinander fest verschraubt, bis sie paßgenau und spielfrei aneinander anliegen.

Der Zielgerätabschnitt 18 ist an seinem dem Befestigungsabschnitt 88 abgewandten Ende 118 um etwa 120° abgewinkelt. Durch dieses verlaufen zwei parallele Zentrierbohrungen 120 und 121, die Längsachsen 122 und 123 definieren, welche den Längsachsen der Querbohrungen 36 und 37 der Knochenplatte 12 entsprechen, wenn diese spielfrei am Zielgerät 10 befestigt ist. Durch die Zentrierbohrungen 120 und 121 können Bohrungen in den Trochanter major 24 des Femurknochens 20 gesetzt werden zum leichteren Einbringen der Knochenschrauben 32 und 33.

Im Bereich seines im Querschnitt rechteckigen Zielabschnitts 94 weist der Zielgerätabschnitt 18 zwei weitere Zentrierbohrungen 124 und 125 auf, die Längsachsen 126 und 127 definieren, die die Längsachsen 122 und 123 der Zentrierbohrungen 120 und 121 unter einem Winkel von etwa 50° schneiden. Die Längsachsen 126 und 127 fallen mit den Längsachsen der Querbohrungen 34 und 35 der Knochenplatte 12 zusammen, wenn diese mit dem Zielgerät 10 spielfrei verbunden ist. Mittels der Zentrierbohrungen 124 und 125 können zum leichteren Einsetzen der Knochenschrauben 30 und 31 in den Femurschaft 22 Bohrungen gesetzt werden.

Zum Einsetzen der Knochenplatte 12 an den Femurknochen 20 wird diese mit dem Zielgerät 10 wie oben erläutert spielfrei mit diesem verbunden. Der Halteabschnitt 14 verlängert die Knochenplatte 12 im wesentlichen in einer Längsrichtung derselben, wobei ein Winkel zwischen den beiden Teilen von maximal 30° eingeschlossen wird. Bei einer vollständig flachen Knochenplatte 12 könnte der Winkel auch 0° betragen. Ein Operateur eröffnet einen Zugang zum menschlichen Körper in einer Größe, der ausreicht, um die Knochenplatte 12 einzuführen. Da der Halteabschnitt 14 keinen größeren Querschnitt als die Knochenplatte 12 aufweist, genügt der kleine Schnitt. Die speziell geformte Knochenplatte 12 verdrängt mit ihrer Einführspitze 44 Haupt- und Körpergewebe, insbesondere Muskeln, und kann so besonders einfach an den Femurknochen 20 in eine gewünschte Position gebracht werden. Zum Fixieren der Knochenplatte 12 werden in Richtung der Längsachsen 122, 123, 125 und 127 kleine Schnitte gesetzt, um zunächst einen Bohrzugang zu schaffen, durch den die Knochenschrauben 30 bis 33 eingeführt und die Knochenplatte 12 mit dem Femurknochen 20 verschraubt werden können.

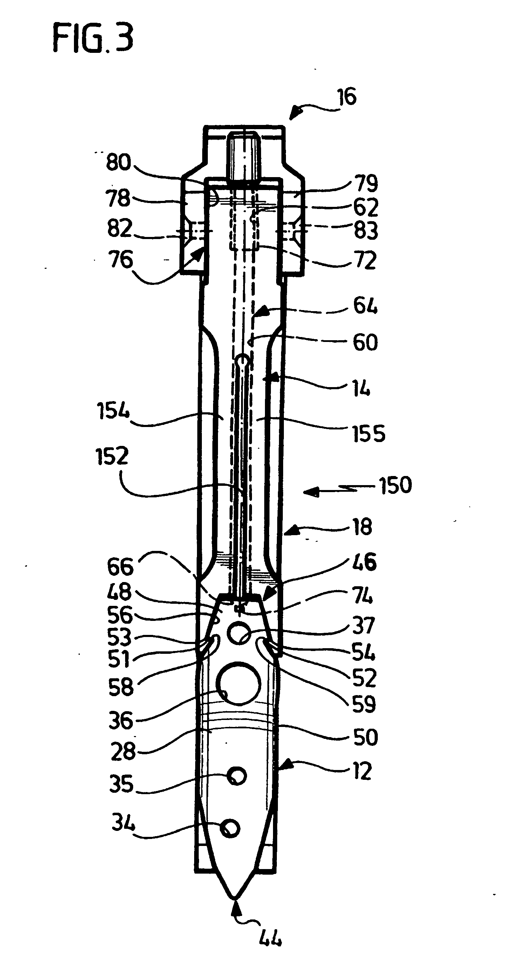

Ein zweites Ausführungsbeispiel eines Zielgeräts ist in Figur 3 dargestellt und insgesamt mit dem Bezugszeichen 150 versehen. Es entspricht im wesentlichen dem Zielgerät 10, bis auf einen Unterschied im Bereich des Halteabschnitts 14. Gleiche Teile sind daher mit denselben Bezugszeichen versehen. Der Halteabschnitt 14 weist einen Längsschlitz 152 auf, der sich in etwa über zwei Drittel der Länge des Halteabschnitts 14 ausgehend von der Plattenaufnahme 56 in Richtung auf das proximale Ende 76 des Halteabschnitts 14 hin erstreckt. Dadurch werden zwei Haltearme 154 und 155 gebildet, die an ihren freien Enden jeweils eine der beiden Rastkanten 58 und 59 tragen.

Das Vorsehen des Längsschlitzes 152 ergibt eine gewisse Biegeelastizität, die es ermöglicht, den Halteabschnitt 14 in Längsrichtung über den Kupplungszapfen 48 der Knochenplatte 12 zu schieben. Außerdem wird dadurch die Knochenplatte zusätzlich quer zur Längsrichtung des Halteabschnitts 14 mit diesem verspannt.

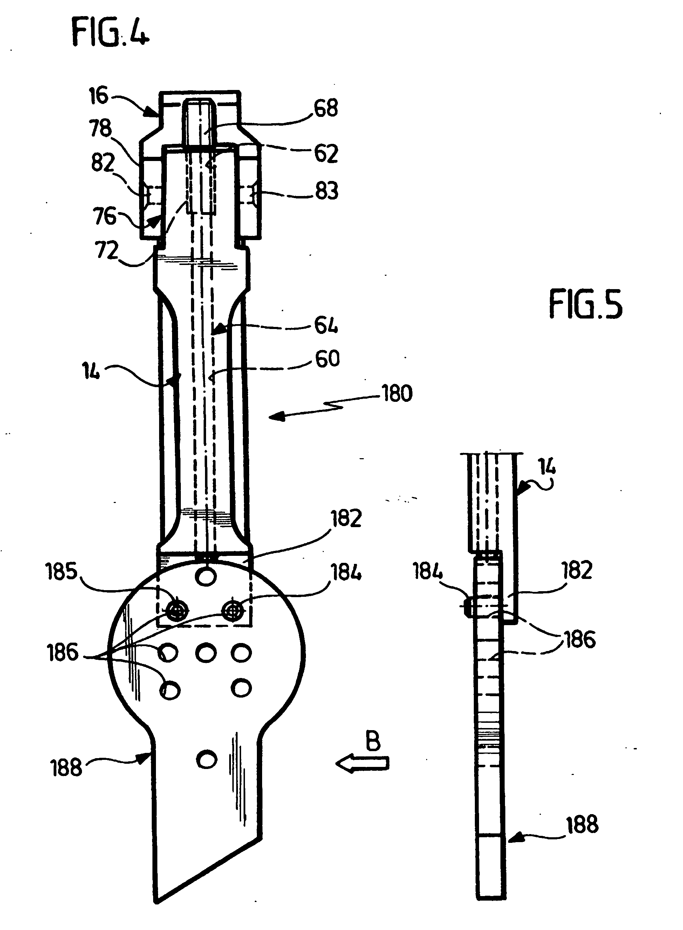

Ein drittes Ausführungsbeispiel eines Zielgeräts ist in den Figuren 4 und 5 insgesamt mit den Bezugszeichen 180 versehen. Es entspricht im wesentlichen dem Zielgerät 10, gleiche Teile sind daher mit denselben Bezugszeichen versehen.

Im Unterschied zum Zielgerät 10 weist der Halteabschnitt 14 des Zielgeräts 180 keine Plattenaufnahme, sondern einen in Längsrichtung des Halteabschnitts abstehenden, flachen, quaderförmigen Plattenhalter 182 auf, von dem zwei Haltestifte 184 und 185 quer zur Längsrichtung des Halteabschnitts 14 abstehen. Die Dicke des Plattenhalters 182 beträgt etwa ein Drittel der Dicke des Halteabschnitts, die Länge der Haltestifte 184 bzw. 185 etwa zwei Drittel der Dicke des Halteabschnitts 14, so daß die Haltestifte 184 und 185 seitlich nicht über den Halteabschnitt 14 hinaus vorstehen.

Die Haltestifte 184 und 185 dienen dazu, korrespondierende Haltebohrungen 186 einer flachen, im wesentlichen ein Schlüsselprofil aufweisenden Kondylenplatte 188 zu durchsetzen.

Die Kondylenplatte 188 kann in Richtung der Längsachsen der Haltestifte 184 und 185 auf diese aufgeschoben werden und mit einem Druckstift 64 wie im Zusammenhang mit den in den Figuren 1 und 2 dargestellten Zielgeräten 10 verspannt werden.

## Patentansprüche

1. Chirurgisches Instrument zum Einsetzen und Halten einer mindestens zwei Knochenteile verbindenden Knochenplatte (12), deren Form eine Einführrichtung definiert, in der die Knochenplatte (12) an die zu verbindenden Knochenteile herangeführt wird, mit einem Halteabschnitt (14), mit mindestens einem an diesem angeordneten Kupplungselement (56; 184, 185) zum lösbaren Verbinden desselben mit mindestens einem an der Knochenplatte (12) vorgesehenen Verbindungselement (48; 186), wobei das Kupplungselement (56; 184, 185) derart angeordnet ist, daß in einer Einführstellung, in der das Instrument (10; 150; 180) mit der Knochenplatte (12) verbunden ist, das Instrument (10; 150; 180) die Knochenplatte (12) im wesentlichen entgegengesetzt zur Einführrichtung verlängert, **dadurch gekennzeichnet, daß** an dem mindestens einen Kupplungselement (56) mindestens ein in der Einführstellung in eine an der Knochenplatte (12) angeordnete, korrespondierende Ausnehmung (51, 52; 186) eintauchender Vorsprung (58, 59; 184, 185) vorgesehen ist.

2. Chirurgisches Instrument nach Anspruch 1, **dadurch gekennzeichnet, daß** das Instrument (10; 150; 180) einen Zielgerätabschnitt (18) mit mindestens einer eine Längsachse (122, 123, 126, 127) aufweisenden Zielgerätdurchbrechung (120, 121, 124, 125) umfaßt und daß der mindestens einen Zielgerätdurchbrechung (120, 121, 124, 125) eine an der Knochenplatte (12) vorgesehene Durchbrechung (34, 35, 36, 37) mit identischer Längsachse (10; 150; 180) zugeordnet ist.

3. Chirurgisches Instrument nach einem der voranstehenden Ansprüche, **dadurch gekennzeichnet, daß** ein Spannglied (64) zum Verspannen der Knochenplatte (12) mit dem Instrument (10; 150; 180) in der Einführstellung vorgesehen ist.

4. Chirurgisches Instrument nach einem der voranstehenden Ansprüche, **dadurch gekennzeichnet, daß** ein mit einem an der Knochenplatte (12) angeordneten Zentrierelement (74) zusammenwirkendes Zentrierglied (64, 66) zum Festlegen einer definierten relativen Kupplungsstellung zwischen dem Instrument (10; 150; 180) und der Knochenplatte (12) in der Einführstellung vorgesehen ist.

5. Chirurgisches Instrument nach Anspruch 4, **dadurch gekennzeichnet, daß** das Zentrierglied (64, 66) einen in Längsrichtung des Halteabschnitts (14) festleg- und auf die Knochenplatte (12) hin bewegbaren Druckstift (64) umfaßt und daß das Zentrierglied (64, 66) ein zum Zentrierelement (74) im wesentlichen korrespondierendes freies Ende (66) aufweist.

6. Chirurgisches Instrument nach einem der Ansprüche 3 bis 5, **dadurch gekennzeichnet, daß** das Spannglied (64) das Zentrierglied (64, 66) umfaßt.

7. Chirurgisches Instrument nach einem der voranstehenden Ansprüche, **dadurch gekennzeichnet, daß** das mindestens eine Kupplungselement (56; 184, 185) mit dem mindestens einen Verbindungselement (48; 186) kraftschlüssig verbindbar ist.

8. Chirurgisches Instrument nach einem der voranstehenden Ansprüche, **dadurch gekennzeichnet, daß** das mindestens eine Kupplungselement (56; 184, 185) mit dem mindestens einen Verbindungselement (48; 186) formschlüssig verbindbar ist.

9. Chirurgisches Instrument nach einem der voranstehenden Ansprüche, **dadurch gekennzeichnet, daß** der mindestens eine Vorsprung durch eine quer zur Einführrichtung verlaufende Rastkante (58, 59) und daß die Ausnehmung durch eine Nut (51, 52) gebildet werden.

10. Chirurgisches Instrument nach einem der voranstehenden Ansprüche, **dadurch gekennzeichnet, daß** der mindestens eine Vorsprung durch einen im wesentlichen quer zur Einführrichtung verlaufenden Stift (184, 185) und daß die Ausnehmung durch eine Durchbrechung (186) der Knochenplatte (12) gebildet wird.

11. Chirurgisches Instrument nach einem der voranstehenden Ansprüche, **dadurch gekennzeichnet, daß** der in der Einführstellung an die Knochenplatte (12) angrenzende Halteabschnitt (14) in einem Übergangsbereich einen Querschnitt aufweist, der höchstens so groß wie der größte Querschnitt der Knochenplatte (12) ist.

12. Chirurgisches Instrument nach einem der voranstehenden Ansprüche, **dadurch gekennzeichnet, daß** der Halteabschnitt (14) zwei Haltearme (154, 155) umfaßt, die sich in Längsrichtung des Halteabschnitts (14) erstrecken, und daß die Haltearme (154, 155) freie Enden aufweisen.

13. Chirurgisches Instrument nach Anspruch 12, **dadurch gekennzeichnet, daß** das mindestens eine Kupplungselement (56) an einem oder an beiden freien Enden der Haltearme (154, 155) angeordnet ist.

14. Chirurgisches Instrument nach einem der Ansprüche 12 oder 13, **dadurch gekennzeichnet, daß** die Haltearme (154, 155) im wesentlichen quer zu ihrer Längsachse biegeelastisch geformt sind.

15. Chirurgisches Instrument nach einem der Ansprüche 2 bis 14, **dadurch gekennzeichnet, daß** mindestens ein Distanzelement (16) vorgesehen ist und daß das Distanzelement (16) den Zielgerätabschnitt (18) und den Halteabschnitt (14) verbindend angeordnet ist.

## Claims

1. A surgical instrument for inserting and holding a bone plate (12) which connects at least two bone parts and the shape of which defines a direction of insertion in which the bone plate (12) is advanced towards the bone parts that are to be connected, said instrument comprising a holding section (14) having at least one coupling element (56; 184, 185) arranged thereon for releasably connecting it to at least one connecting element (48; 186) provided on the bone plate (12), wherein the coupling element (56; 184, 185) is arranged in such a manner that in an insertion position in which the instrument (10; 150; 180) is connected to the bone plate (12), the instrument (10; 150; 180) elongates the bone plate (12) in a direction substantially opposed to the direction of insertion, **characterised in that** at least one projection (58, 59; 184, 185) which enters a corresponding recess (51, 52; 186) arranged in the bone plate (12) in the insertion position is provided on the at least one coupling element (56).

2. A surgical instrument in accordance with Claim 1, **characterised in that** the instrument (10; 150; 180) comprises a target device section (18) incorporating at least one target device through-opening (120, 121, 124, 125) having a longitudinal axis (122, 123, 126, 127), and **in that** a through-opening (34, 35, 36, 37) having an identical longitudinal axis (10; 150; 180) that is provided in the bone plate (12) is associated with the at least one target device through-opening (120, 121, 124, 125).

3. A surgical instrument in accordance with any of the preceding Claims, **characterised in that** there is provided a clamping member (64) for clamping the bone plate (12) to the instrument (10; 150; 180) in the insertion position.

4. A surgical instrument in accordance with any of the preceding Claims, **characterised in that** a centring member (64, 66) cooperating with a centring element (74) arranged on the bone plate (12) is provided for establishing a defined relative coupling position between the instrument (10; 150; 180) and the bone plate (12) in the insertion position.

5. A surgical instrument in accordance with Claim 4, **characterised in that** the centring member (64, 66) comprises a pressure pin (64) which can be fixed in the longitudinal direction of the holding section (14) and can be moved towards the bone plate (12), and **in that** the centring member (64, 66) has a free end (66) substantially corresponding to the centring element (74).

6. A surgical instrument in accordance with any of the Claims 3 to 5, **characterised in that** the clamping member (64) comprises the centring member (64, 66).

7. A surgical instrument in accordance with any of the preceding Claims, **characterised in that** the at least one coupling element (56; 184, 185) can be connected to the at least one connecting element (48; 186) in force-transmitting manner.

8. A surgical instrument in accordance with any of the preceding Claims, **characterised in that** the at least one coupling element (56; 184, 185) can be connected to the at least one connecting element (48; 186) in positive manner.

9. A surgical instrument in accordance with any of the preceding Claims, **characterised in that** the at least one projection is formed by a latching edge (58, 59) running transversely to the direction of insertion and **in that** the recess is formed by a groove (51, 52).

10. A surgical instrument in accordance with any of the preceding Claims, **characterised in that** the at least one projection is formed by a pin (184, 185) running substantially transversely relative to the direction of insertion, and **in that** the recess is formed by a through-opening (186) in the bone plate (12).

11. A surgical instrument in accordance with any of the preceding Claims, **characterised in that** the cross section of a transitional region of the holding section (14) bordering the bone plate (12) in the insertion position is at most as large as the largest cross section of the bone plate (12).

12. A surgical instrument in accordance with any of the preceding Claims, **characterised in that** the holding section (14) comprises two holding arms (154, 155) which extend in the longitudinal direction of the holding section (14), and **in that** the holding arms (154, 155) have free ends.

13. A surgical instrument in accordance with Claim 12, **characterised in that** the at least one coupling element (56) is arranged at one or at both free ends of the holding arms (154, 155).

14. A surgical instrument in accordance with either of the Claims 12 or 13, **characterised in that** the holding arms (154, 155) are flexural in a direction substantially transverse to the longitudinal axis thereof.

15. A surgical instrument in accordance with any of the Claims 2 to 14, **characterised in that** at least one distance element (16) is provided, and **in that** the distance element (16) is arranged in a manner connecting the target device section (18) and the holding section (14).

## Revendications

1. Instrument chirurgical pour mettre en place et retenir une plaque osseuse (12) reliant au moins deux parties d'os, dont la forme définit une direction d'introduction dans laquelle la plaque osseuse (12) est amenée aux parties d'os à relier, comportant un tronçon de retenue (14), au moins un élément d'accouplement (56; 184, 185) agencé sur celui-ci pour relier ce dernier de manière détachable avec au moins un élément de liaison (48; 186) prévu sur la plaque osseuse (12), l'élément d'accouplement (56; 184, 185) étant agencé de telle sorte que dans une position d'introduction, dans laquelle l'instrument (10 ; 150 ; 180) est relié à la plaque osseuse (12), l'instrument (10; 150; 180) prolonge la plaque osseuse (12) sensiblement à l'opposé de la direction d'introduction, **caractérisé en ce que** sur ledit au moins un élément d'accouplement (56) est prévue au moins une saillie (58, 59, 184, 185) plongeant, dans la position d'introduction, dans un évidement (51, 52; 186) correspondant ménagé sur la plaque osseuse (12).

2. Instrument chirurgical selon la revendication 1, **caractérisé en ce que** l'instrument (10; 50; 180) comprend un tronçon de viseur (18) avec au moins une percée de viseur (120, 121, 124, 125) présentant un axe longitudinal (122, 123, 126, 127), et **en ce qu'**à ladite au moins une percée de viseur (120, 121, 124, 125) est associée une percée (34, 35, 36, 37) prévue sur la plaque osseuse (12) avec un axe longitudinal (10; 150; 180) identique.

3. Instrument chirurgical selon l'une des revendications précédentes, **caractérisé en ce qu'**il est prévu un organe de serrage (64) pour serrer la plaque osseuse (12) avec l'instrument (10; 150; 180) dans la position d'introduction.

4. Instrument chirurgical selon l'une des revendications précédentes, **caractérisé en ce qu'**il est prévu un organe de centrage (64, 66) coopérant avec un élément de centrage (74) agencé sur la plaque osseuse (12) pour immobiliser une position d'accouplement relative définie entre l'instrument (10; 150; 180) et la plaque osseuse (12) dans la position d'introduction.

5. Instrument chirurgical selon la revendication 4, **caractérisé en ce que** l'organe de centrage (64, 66) comprend une tige de pression (64) immobilisable en direction longitudinale du tronçon de retenue (12) et déplaçable en direction de la plaque osseuse (12), et **en ce que** l'organe de centrage (64, 66) présente une extrémité libre (66) correspondant sensiblement à l'élément de centrage (74).

6. Instrument chirurgical selon l'une des revendications 3 à 5, **caractérisé en ce que** l'organe de serrage (64) comprend l'organe de centrage (64, 66).

7. Instrument chirurgical selon l'une des revendications précédentes, **caractérisé en ce que** ledit au moins un élément d'accouplement (56 ; 184, 185) peut être relié par coopération de forces avec ledit au moins un élément de liaison (48; 186).

8. Instrument chirurgical selon l'une des revendications précédentes, **caractérisé en ce que** ledit au moins un élément d'accouplement (56 ; 184, 185) peut être relié par coopération de formes avec ledit au moins un élément de liaison (48; 186).

9. Instrument chirurgical selon l'une des revendications précédentes, **caractérisé en ce que** ladite au moins une saillie est formée par une arête d'enclenchement (58, 59) s'étendant transversalement à la direction d'introduction, et **en ce que** l'évidement est formé par une gorge (51, 52).

10. Instrument chirurgical selon l'une des revendications précédentes, **caractérisé en ce que** ladite au moins une saillie est formée par une goupille (184, 185) s'étendant sensiblement transversalement à la direction d'introduction, et **en ce que** l'évidement est formé par une percée (186) de la plaque osseuse (12).

11. Instrument chirurgical selon l'une des revendications précédentes, **caractérisé en ce que** le tronçon de retenue (14) adjacent à la plaque osseuse (12) dans la position d'introduction présente dans une région de transition une section transversale qui est au plus aussi grande que la plus grande section transversale de la plaque osseuse (12).

12. Instrument chirurgical selon l'une des revendications précédentes, **caractérisé en ce que** le tronçon de retenue (14) comprend deux bras de retenue (154, 155) qui s'étendent en direction longitudinale du tronçon de retenue (14), et **en ce que** les bras de retenue (154, 155) présentent des extrémités libres.

13. Instrument chirurgical selon la revendication 12, **caractérisé en ce que** ledit au moins un élément d'accouplement (56) est agencé sur une ou sur les deux extrémités libres des bras de retenue (154, 155).

14. Instrument chirurgical selon l'une des revendications 12 ou 13, **caractérisé en ce que** les bras de retenue (154, 155) sont formés sensiblement élastiques à la flexion transversalement à leur axe longitudinal.

15. Instrument chirurgical selon l'une des revendications 2 à 14, **caractérisé en ce qu'**il est prévu au moins un espaceur (16) et **en ce que** l'espaceur (16) est agencé en reliant le tronçon de viseur (18) et le tronçon de retenue (14).
